# EUROPEAN PATENT SPECIFICATION

(11) **EP 4 072 616 B1**
(45) Date of publication and mention of the grant of the patent: **16.04.2025**
(21) Application number: 20830443.6
(22) Date of filing: 07.12.2020
(51) Int. Cl.: A61M 39/10, A61M 39/08, A61M 5/14, F16L 11/12, F16L 33/34, G01M 3/22

(54) **TUBING MARKERS**
SCHLAUCHMARKER
MARQUEURS DE TUBE

(30) Priority: 09.12.2019 US 201916708075
(43) Date of publication of application: 19.10.2022
(73) Proprietor: Carefusion 303 Inc., San Diego, California 92130 (US)
(72) Inventor: SUWITO, Wantjinarjo, Irvine, California 92604 (US)
(74) Representative: Epping - Hermann - Fischer
(86) International application number: PCT/US2020/063661
(87) International publication number: WO 2021/118939

(56) References cited:
- WO-A1-2013/032767
- IT-A1- UB20 154 088
- JP-A- 2006 234 117
- US-A1- 2016 199 569
- US-A1- 2017 321 825

## Description

### BACKGROUND

Infusion or intravenous (IV) sets are constructed by joining multiple translucent polymeric tubing segments to multiple polymeric components that are either translucent or opaque. The joints are typically formed by applying a thin layer of solvent or adhesive on one or both of the contacting surfaces and then the two surfaces are brought together. The contacting surface of the tubing can either be at the inner diameter, the outer diameter, or both diameters up to a certain length from one end. The solvent/adhesive is applied either internally, externally, or both. The bonded area, or the bond length for a given diameter, is a critical parameter that if not controlled can cause the infusion sets to leak or separate easily. The bond parameter can vary significantly due to design shortfalls, assembly process shortfalls and process drifts. Document IT UB20 154 088 A1 discloses an assembly process of a flexible hose to a connector by means of a clamping sleeve. The method comprises affixing a marking at the hose in proximity of at least one end of the hose intended to be associated with a clamping sleeve. The method further comprises inserting a tube into the clamping sleeve and detect the position of the marking with respect to the clamping sleeve. On the basis of the detected position of the marking the coupling of the flexible tube with the clamping sleeve can be rejected as defective, or accepted as good.

Document US 2017/321825 A1 discloses a tubing including insertion reference markings on its outer surface that provide an indicator of how far the tubing is inserted into a fitting. There can be a first insertion reference marking and a second insertion reference marking and distance between these first and second insertion reference marking is a predetermined distance. The documents JP 2006 234117 A, US 2016/199569 A1 and WO 2013/032767 A1 discloses further tubing configurations. The WO 2013/032767 A1 document is related to the inspection of bonded joints and, in particular, to bonded joints between tubes and fittings in medical systems. A joining material is applied to one of the overlapping surfaces of a tube and a fitting into which the tube is to be inserted. The joining material is coloured such that an operator or a machine-vision system can visually inspect the joint by observing the bonding area. The joining material may alternatively be a material that responds to non-visible radiation, such as ultraviolet light.

Inspecting this critical bond length can be done by visual inspection of the solvent/adhesive wetted area along the contacting surfaces. Another method is inspecting the gap between the tubing end and a hard stop within the component. Both require inspections through translucent components. However, if the component is wavy, textured or opaque, then a visual inspection is difficult to perform, or it cannot be done at all. Further, in some situations a visible marker is not desirable, such as for commercial products intended for the consumer market. It is desirable to provide for reliable inspections using invisible markers for all infusion set construction regardless of translucency of the components to minimize leaks, separations and variations in total infusion set length.

### SUMMARY

The present disclosure provides an intravenous tube for use in an infusion set as set out in the appended set of claims. The present disclosure further provides an infusion set assembly as set out in the appended set of claims. The present disclosure provides invisible markers for tubing used in infusion sets to provide a visual indicator of the fit between an infusion tube and an infusion set component when subjected to a particular source, such as Ultraviolet (UV) light.

In one or more embodiments, a tube for use in an infusion set is provided. The tube includes a body defining a fluid flow pathway, a first tube end and a second tube end. A tube marker is disposed near the first tube end. The tube marker is positioned so that a distance between a leading edge of the tube marker and the outermost surface of the first tube end is substantially equal to a distance between an outermost surface of a fluid port of an infusion set component and a hard stop of the fluid port. The tube marker comprises material that is only visible under a specific light spectrum.

In one or more aspects, the specific light spectrum is UV light provided by a UV light source. In one or more aspects, the first tube end is a fluid input end configured to be inserted into a fluid outlet port of the infusion set component. In one or more aspects, the first tube end is a fluid output end configured to be inserted into a fluid inlet port of the infusion set component. According to the invention, the tube marker includes one or more cylindrical bands. In one or more aspects, the tube marker material includes printed ink. In one or more aspects, the printed ink comprises one of a UV dye and a UV ink.

According to the invention, the tube includes a second tube marker disposed near the second tube end, wherein the second tube marker is positioned so that a distance between a leading edge of the second tube marker and the outermost surface of the second tube end is substantially equal to a distance between an outermost surface of a second infusion set component fluid port and a hard stop of the second infusion set component fluid port. In one or more aspects, the second tube marker includes a material that is only visible under the same specific light spectrum as the first tube marker. In one or more aspects, the second tube marker includes a material that is different than the material of the first tube marker, and wherein the second tube marker is only visible under a different specific light spectrum than is the first tube marker. In one or more aspects, the first tube end is a fluid input end configured to be inserted into a fluid outlet port of the first infusion set component and the second tube end is a fluid output end configured to be inserted into a fluid inlet port of the second infusion set component. In one or more aspects, the first tube end is a fluid output end configured to be inserted into a fluid inlet port of the first infusion set component and the second tube end is a fluid input end configured to be inserted into a fluid outlet port of the second infusion set component.

In one or more embodiments, an infusion set assembly is provided. The infusion set assembly includes an infusion component and a tube coupled to the infusion component. The tube includes a body defining a fluid flow pathway, a first tube end, a second tube end and a tube marker including material that is only visible under a specific light spectrum. The tube marker is positioned near the first tube end so that a distance between a leading edge of the tube marker and the outermost surface of the first tube end is substantially equal to a distance between an external edge and a hard stop of a fluid port of the infusion component.

In one or more aspects, the tube marker material includes one of UV ink and UV dye and the specific light spectrum is UV light provided by a UV light source. In one or more aspects, the tube marker includes at least one cylindrical band formed by an inkjet printer. In one or more aspects, the infusion set assembly includes a second infusion component and a second tube marker including the material that is only visible under the specific light spectrum and that is disposed near the second tube end. The second tube marker is positioned so that a distance between a leading edge of the second tube marker and the outermost surface of the second tube end is substantially equal to a distance between an external edge and a hard stop of a fluid port of the second infusion component. In one or more aspects, the infusion set assembly include one of a solvent and an adhesive having securing material that is only visible under the specific light spectrum. In one or more aspects, the securing material includes a UV dye.

Additional features and advantages of the disclosure will be set forth in the description below and, in part, will be apparent from the description or may be learned by practice of the disclosure. The objectives and other advantages of the disclosure will be realized and attained by the structure particularly pointed out in the written description and claims hereof as well as the appended drawings.

It is to be understood that both the foregoing general description and the following detailed description are exemplary and explanatory and are intended to provide further explanation of the disclosure as claimed.

### BRIEF DESCRIPTION OF THE DRAWINGS

The accompanying drawings, which are included to provide further understanding of the disclosure and are incorporated in and constitute a part of this specification, illustrate embodiments of the disclosure and together with the description serve to explain the principles of the disclosure.
FIG. 1 depicts a schematic view of a typical assembled infusion set.
FIG. 2 depicts a schematic view of a typical infusion set component and tubing prior to assembly.
FIG. 3 depicts a cross-section view of the infusion set component of FIG. 2.
FIG. 4 depicts a schematic view of the infusion set component and tubing of FIG. 2 after assembly.
FIG. 5 depicts a cross-section view of the infusion set component and tubing of FIG. 4.
FIG. 6 depicts a schematic view of one or more embodiments of an assembled infusion set component and tubing with markers made visible by specific lighting.
FIG. 7 depicts a cross-sectional view of the infusion set component of FIG. 6.
FIG. 8 depicts a schematic view of one or more embodiments of a tubing segment with markers made visible by specific lighting.
FIG. 9 depicts a schematic view of one or more embodiments of a tubing segment with markers made visible by specific lighting.
FIG. 10 depicts an enlarged view of a portion of the tubing segment of FIG. 9.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions are provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Infusion sets may be formed from any combination of infusion components and tubing. Typically, the infusion components and tubing are disposable products that are used once and then discarded. The infusion components and tubing may be formed from any suitable material (e.g., plastic, silicone, rubber). An issue in manufacturing infusion sets is consistently joining the tubing and the infusion components to obtain a secure and/or leak free joint with desired fluid flow. The joint may be formed at either the inner or outer diameter of the tubing. In some cases, the joint may be formed both by bonding inner and outer diameters of the tubing to a component for additional joint strength.

Prior approaches provide a partial solution by making the infusion components translucent. However, even inspecting through a translucent body can be challenging when the outer surface is not smooth or additional layers of material are present, such as a textured gripping surface. In some cases, the infusion components are made opaque and with different colors to indicate a fluid flow direction (e.g., a check valve). Since seeing through opaque material is difficult to impossible, another prior approach is that the joint is typically made at the inner diameter of the tubing. This restricts fluid flow due to a smaller orifice than the tubing inner diameter at the joint. Additionally, applying solvent/adhesive on the inside of the tubing has a higher propensity for occlusions, because the solvent/adhesive is pushed in as the tube is inserted to form a joint.

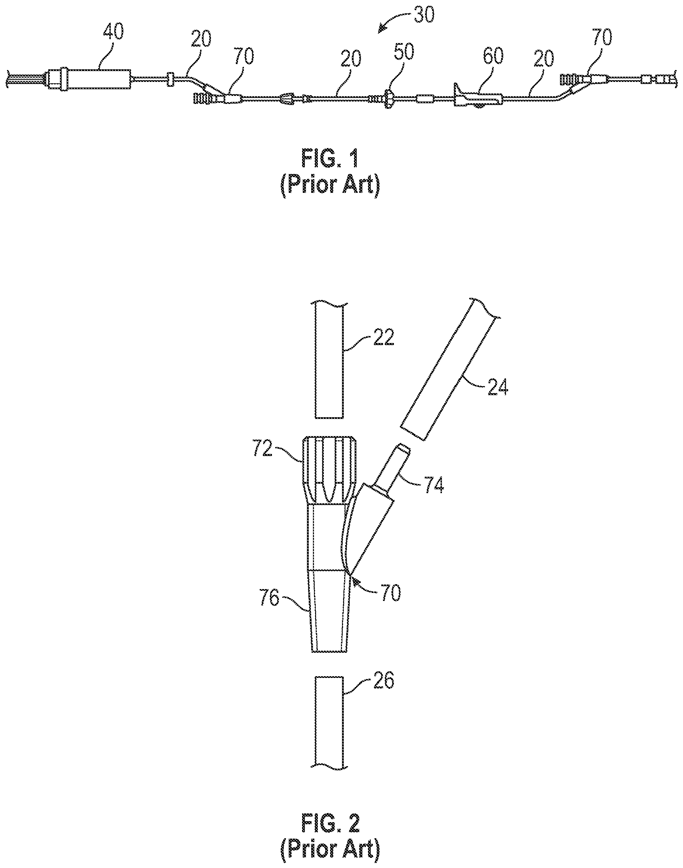

As shown in FIG. 1, a typical infusion set 30 may include a drip chamber 40, a check valve 50, a roller clamp 60 and Y-junctions 70, all connected together by tubing 20. A typical infusion set 30 can include additional infusion components and can be formed of any combination of components and the tubing 20.

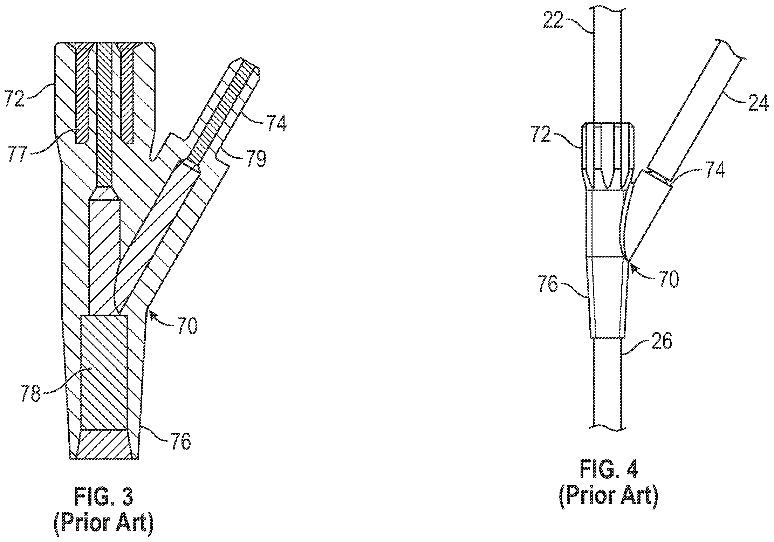

As shown in FIG. 2, a typical Y-junction 70 has inlet ports 72, 74 and an outlet port 76, where each inlet port 72, 74 is to be connected to an inlet tube 22, 24 and the outlet port 76 is to be connected to an outlet tube 26. As shown in FIG. 3, the inlet port 72 has an internal joint 77 where the inlet tube 22 is to be inserted into the inlet port 72. Similarly, the outlet port 76 has an internal joint 78 where the outlet tube 26 is to be inserted into the outlet port. 76. By contrast, the inlet port 74 has an external joint 79 where the inlet tube 24 is to be slid onto and over the inlet port 74.

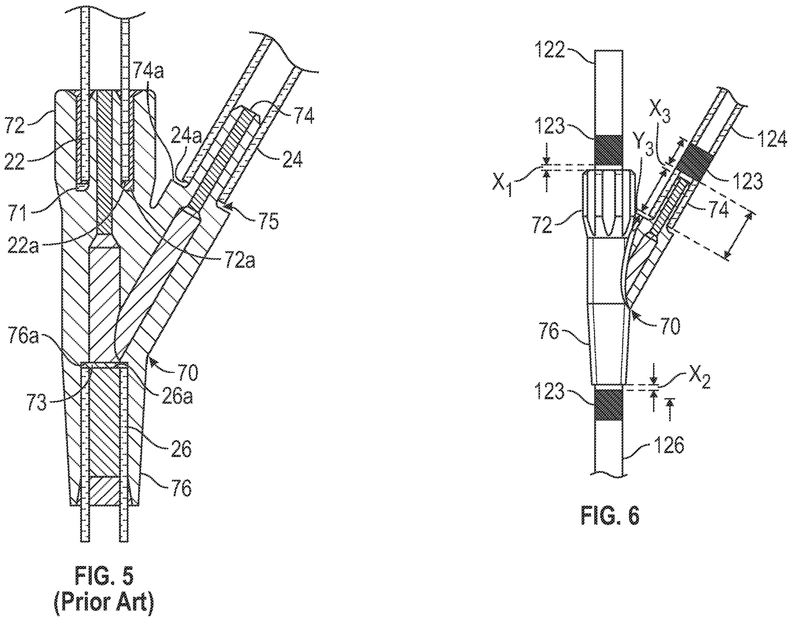

FIGS. 4 and 5 show the Y-junction 70 and the inlet/outlet tubes 22, 24, 26 after assembly. An incomplete joining of the inlet tube 22 with the inlet port 72. leaves a gap 71 between an end surface 22a of the inlet tube 22 and an engagement surface 72a of the inlet port 72. Similarly, an incomplete joining of the outlet tube 26 with the outlet port 76 leaves a gap 73 between an end surface 26a of the outlet tube 26 and an engagement surface 76a of the outlet port 76. Further, an incomplete joining of the inlet tube 24 with the inlet port 74 leaves a gap 75 between an end surface 24a of the inlet tube 24 and an engagement surface 74a of the inlet port 74. As discussed above, the gaps 71, 73, 75 increase the likelihood that the respective internal/external joints 77, 78, 79 will fail, either by leaking or by separation of the inlet/outlet tubes 22, 24, 26 from the Y-junction 70.

Unobstructed visual inspections on sharp edges with good contrast lend themselves for good repeatability and reproducibility (R&R). This is more so for automated computer vision inspections. An acceptable gage R&R is required for measuring critical parameters, such as the case for bond joint area/length. This critical parameter is currently measured by measuring the gap between the tubing end and the insertion hard stop within the component. This method has been found difficult to do in some cases and impossible in others.

By adding markers or marker bands along the tubing that are invisible under typical lighting but visible under a specific light spectrum or light source (e.g., UV light, infrared light), an element to make a good distance measurement is provided. The infusion components are typically injection molded with great dimensional precision. The distance between the hard stop and an external edge can generally be deduced from a drawing. Therefore, the distance between the marker (e.g., UV marker) and that external edge can be used to deduce the bond length.

The marker bands or markers may be produced by a pad printing method, laser marking, or any other suitable method, as long as good contrasting markers under the correct light source are produced. For example, a UV ink may be printed onto the tubing to produce markers that become visible (e.g., fluoresce) when subjected to UV light. As another example, a UV dye may be combined with or mixed into the solvent or adhesive used to join the tubing and the components, where the areas of the tubing and component containing the combined solvent/adhesive become visible when subjected to UV light.

The markers may be high contrasting solid bands under appropriate lighting. The bands may be two or more fragmented blobs at the outer diameter of tubing. The shape may be a band or any other suitable shape that can be easily seen under the specific light source and analyzed with unaided eyes or computer vision, as well as does not block fluid flow inspection. For example, visible ink markers on a translucent tubing may need to be less than a particular fraction of the tubing surface area to not block fluid flow inspection. Invisible ink markers (e.g., UV markers), however, may take up to 100% of the tubing surface area while not blocking any fluid flow inspection at all under normal lighting conditions. Thus, manufacturers may subject invisible markers to an appropriate light source (e.g., UV markers subjected to UV light) to verify that bond joints are fully inserted to ensure reliable joints and infusion/IV sets, while downstream supply chain users (e.g., health care providers, patients, consumers) may use the tubing/IV set under normal lighting without visible markings that distract or block visual inspection during use (e.g., fluid flow inspection).

For example, invisible markers may be UV markers formed using UV dye and chemicals that, once applied to tubing, become biocompatible and do not smear with the close proximity of solvent used in bonding. The UV markers can fluoresce using a low power UV light source while not affecting the health of the operators (e.g., health care providers, patients), and with proper protections (e.g., storage, handling) may be safe to use for a long period of time.

Markers on a tube may all be formed from the same invisible marking ink or process. Here, only one special light source is needed at the manufacturing process to identify all of the markers. As another example, some markers may be formed to be visible under one special light source (e.g., UV light) and other markers may be formed to be visible under a different special light source (e.g., infrared). In such a manner, one set of markers may be utilized in one portion of the manufacturing process and the other set of markers may be utilized in another portion of the manufacturing process, while both sets of markers remain invisible under typical lighting conditions.

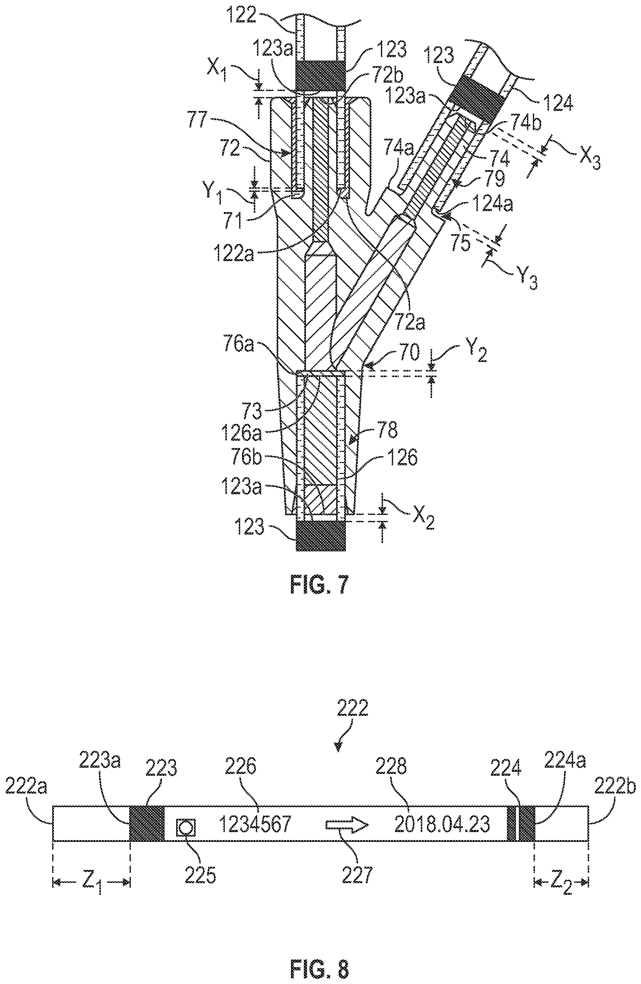

As shown in FIGS. 6 and 7, a Y-joint 70 may be joined together with inlet tubes 122, 124 and outlet tube 126. The inlet tube 122 includes a tube marker 123 visible under particular lighting (e.g., UV light), which is disposed at a specific position on the inlet tube 122. Thus, when a leading edge 123a of the tube mark 123 is aligned with (e.g., even with) the top surface 72b of the inlet port 122, there is no gap between the end surface 122a of the inlet tube 122 and the engagement surface 72a of the inlet port 72 in the internal joint 77. As another example, if there is a gap 71 between the end surface 122a of the inlet tube 122 and the engagement surface 72a of the inlet port 72, the distance x₁ between the leading edge 123a of the tube mark 123 and the top surface 72b of the inlet port 72 is commensurate or substantially equal to the distance y₁ of gap 71.

Similarly, for the internal joint 78 formed by the outlet tube 126 and the outlet port 76, the outlet tube 126 includes the tube marker 123 disposed at a specific position on the outlet tube 126 and visible under UV light. Here, when a leading edge 123a of the tube mark 123 is aligned with (e.g., even with) the bottom surface 76b of the outlet port 76, there is no gap between the end surface 126a. of the outlet tube 126 and the engagement surface 76a of the outlet port 76. If there is a gap 73 between the end surface 126a of the outlet tube 126 and the engagement surface 76a of the outlet port 76, the distance x₂ between the leading edge 123a of the tube mark 123 and the bottom surface 76b of the outlet port 76 is commensurate or substantially equal to the distance y₂ of gap 73.

For the external joint 79 formed by the inlet tube 124 and the inlet port 74, the inlet tube 124 includes the tube marker 123 disposed at a specific position on the inlet tube 124 and visible under UV light. When a leading edge 123a of the tube mark 123 is aligned with (e.g., even with) the top surface 74b of the outlet port 74, there is no gap between the end surface 124a of the inlet tube 124 and the engagement surface 74a of the inlet port 74. If there is a gap 75 between the end surface 124a of the inlet tube 124 and the engagement surface 74a of the inlet port 74, the distance x₃ between the leading edge 123a of the tube mark 123 and the top surface 74b of the inlet port 74 is commensurate or substantially equal to the distance y₃ of gap 75.

As shown in FIG. 8, a tube 222 may include markers 223, 224 at both ends for providing visual guidance under specific lighting (e.g., UV light) during joining of the tube 222 to infusion set components. The markers 223, 224 may have different characteristics or looks based on different marker distances from the tube ends. For example, the marker 223 may be a single solid band that is associated with a distance z₁ between the leading edge 223a of the marker 223 and the first tube end 222a. As another example, the marker 224 may be two different sized solid bands that are associated with a distance z₂ between the leading edge 224a of the marker 224 and the second tube end 222b. The tube 222 may also include one or more informational markings, such as a logo 225, a part number/unique device identification 226, a symbol 227 and a date code 228, any or all of which may be visible under normal lighting or only visible under specific lighting (e.g., UV light). Informational markings visible under normal lighting may be disposed between the markers 223, 224 so that the portions of the tube 222 from the leading edge of the markers 223a, 224a to the tube ends 222a, 222b remain clear of markings for ease of use of markers 223, 224. Informational markings visible only under special lighting may be disposed anywhere on the tube 222 without providing a distraction or blocking views inside the tube 222 under normal lighting conditions. The informational markings may provide relevant information regarding the tubing 222, the infusion set components and/or the infusion set. Thus, the relevant information may be easily discerned from the tubing 222 under appropriate lighting instead of having to be determined from the associated packaging that is typically discarded.

Another related aspect of the disclosure is determining when and where to add the markers. Infusion set tubing is typically produced using an extrusion method and spooled. The spooled tubing is then cut to desired lengths and the cut tubing is then joined to infusion set components to make an infusion set. The present disclosure provides for a marking tool (e.g., pad printer, laser) to be disposed in a cutting station in which the tubing may be cut to a precise length and markers may be applied at the same cutting and marking machine/station. Thus, no additional process time is incurred by moving materials, loading materials and adding the markers on a different machine/station.

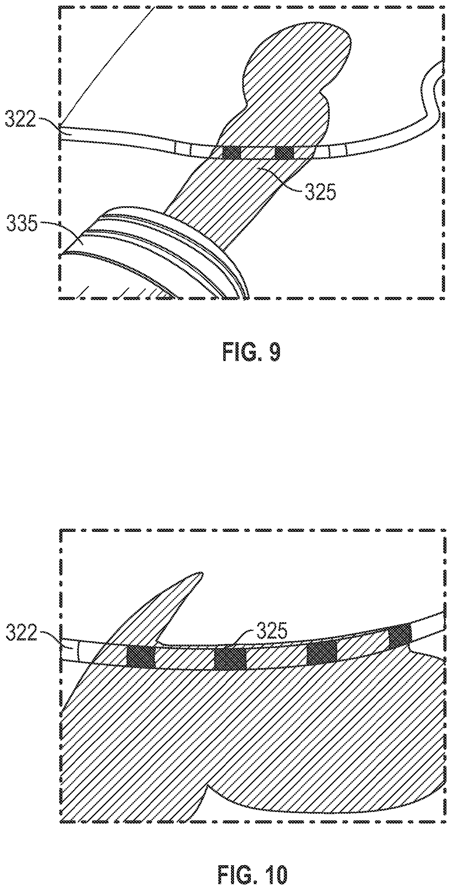

As shown in FIG. 9, a tube 322 may include markers 325 disposed periodically along the tube 322 for providing visual guidance under a special light source 335 (e.g., UV light). For example, the visual guidance may be utilized during joining of the tube 322 to infusion set components. The markers 325 may be formed on the tube 322 by a printer using special ink that is not visible under typical lighting conditions (e.g., sunlight, incandescent light, fluorescent light) and is visible under light from the special light source 335. For example, a UV dye may be added to standard printer ink, or a specific manufactured UV ink, may be used in a standard printer used to print markings on tubing such as the tube 322. The markers 325 may be formed as patterns of inkjet dots as shown in FIG. 10. As discussed above, the markers 325 may be formed by any desired printing technology (e.g., laser printing, thermal printing).

It is understood that any specific order or hierarchy of blocks in the methods of processes disclosed is an illustration of example approaches. Based upon design or implementation preferences, it is understood that the specific order or hierarchy of blocks in the processes may be rearranged, or that all illustrated blocks be performed. In some implementations, any of the blocks may be performed simultaneously.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of ' preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect. may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, operations or processes disclosed is an illustration of exemplary approaches. Based upon design preferences, it is understood that the specific order or hierarchy of steps, operations or processes may be rearranged. Some of the steps, operations or processes may be performed simultaneously. Some or all of the steps, operations, or processes may be performed automatically, without the intervention of a user.

All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

## Claims

1. An intravenous tube (122, 123, 124, 222, 322) for use in an infusion set (30), the tube (122, 123, 124, 222, 322) comprising:
a flexible polymeric body defining a fluid flow pathway;
a first tube end (122, 222a);
a second tube end (124, 126, 222b);
a first tube marker (123, 223, 224, 325) disposed on the tube (122, 123, 124, 222, 322), wherein the first tube marker (123, 223, 224, 325) is positioned so that a distance (z1) between a leading edge (123a, 223a, 224a) of the first tube marker (123, 223, 224, 325) and an outermost surface (122a) of the first tube end (122, 222a) is equal to a distance between an outermost surface of a fluid port (72, 74, 76) of a first infusion set component (40, 50, 60, 70) and a hard stop of the fluid port (72, 74, 76), wherein the hard stop is configured to contact and block the outermost surface (122a) of the first tube end (122, 222a) from further advancement into the fluid port (72, 74, 76) of the first infusion set component (40, 50, 60, 70), wherein the first tube marker (123, 223, 224, 325) comprises material that is only visible under an ultraviolet, UV, light spectrum, and wherein the first tube marker (123, 223, 224, 325) comprises multiple cylindrical bands disposed on the first tube end (122, 222a);
a second tube marker (123, 223, 224, 325) disposed on the tube (122, 123, 124, 222, 322), wherein the second tube marker (123, 223, 224, 325) is positioned so that a distance (z2) between a leading edge (123a, 223a, 224a) of the second tube marker (123, 223, 224, 325) and the outermost surface (122a) of the second tube end (124, 126, 222b) is substantially equal to a distance between an outermost surface of a second infusion set component fluid port (72, 74, 76) and a hard stop of the second infusion set component fluid port (72, 74, 76), wherein the second tube marker (123, 223, 224, 325) comprises material that is only visible under a specific light spectrum, and wherein the second tube marker (123, 223, 224, 325) comprises a different number of cylindrical bands than the first tube marker (123, 223, 224, 325); and
an informational marking (225, 226, 227, 228) disposed on the tube (122, 123, 124, 222, 322), wherein the informational marking (225, 226, 227, 228) comprises material that is only visible under a UV light spectrum.

2. The tube (122, 123, 124, 222, 322) of Claim 1, wherein the multiple cylindrical bands comprise two different sized solid bands.

3. The tube (122, 123, 124, 222, 322) of Claim 1, wherein the tube marker material comprises printed ink.

4. The tube (122, 123, 124, 222, 322) of Claim 3, wherein the printed ink comprises a UV dye.

5. The tube (122, 123, 124, 222, 322) of Claim 3, wherein the printed ink comprises a UV ink.

6. The tube (122, 123, 124, 222, 322) of Claim 1, wherein the second tube marker (123, 223, 224, 325) comprises a material that is only visible under the same UV light spectrum as the first tube marker (123, 223, 224, 325).

7. The tube (122, 123, 124, 222, 322) of Claim 1, wherein the second tube marker (123, 223, 224, 325) comprises a material that is different than the material of the first tube marker (123, 223, 224, 325), and wherein the second tube marker (123, 223, 224, 325) is only visible under a different specific light spectrum than is the first tube marker (123, 223, 224, 325).

8. An infusion set assembly (30), comprising:
an infusion component (40, 50, 60, 70); and
the tube (122, 123, 124, 222, 322) of any of the preceding claims coupled to the infusion component (40, 50, 60, 70).

9. The infusion set assembly (30) of Claim 8, wherein the tube marker material comprises one of UV ink and UV dye.

10. The infusion set assembly (30) of Claim 8, wherein the multiple cylindrical bands of the first tube marker (123, 223, 224, 325) are formed by an inkjet printer.

11. The infusion set assembly (30) of Claim 8, further comprising:
a second infusion component (40, 50, 60, 70), wherein the second tube marker (123, 223, 224, 325) is positioned so that a distance (z2) between a leading edge (123a, 223a, 224a) of the second tube marker (123, 223, 224, 325) and the outermost surface of the second tube end (124, 126, 222b) is equal to a distance between an external edge and a hard stop of a fluid port of the second infusion component (40, 50, 60, 70).

12. The infusion set assembly (30) of Claim 8, further comprising:
one of a solvent and an adhesive comprising securing material joining the first tube end (122, 222a) to the infusion component (40, 50, 60, 70) and that is only visible under the UV light spectrum.

13. The infusion set assembly (30) of Claim 12, wherein the securing material comprises a UV dye.

## Patentansprüche

1. Intravenöser Schlauch (122, 123, 124, 222, 322) zur Verwendung in einem Infusionsset (30), wobei der Schlauch (122, 123, 124, 222, 322) umfasst:
einen flexiblen Polymerkörper, der einen Flüssigkeitsdurchflussweg definiert;
ein erstes Schlauchende (122, 222a);
ein zweites Schlauchende (124, 126, 222b);
eine erste Schlauchmarkierung (123, 223, 224, 325), die auf dem Schlauch (122, 123, 124, 222, 322) angeordnet ist, wobei die erste Schlauchmarkierung (123, 223, 224, 325) so positioniert ist, dass ein Abstand (z1) zwischen einer Vorderkante (123a, 223a, 224a) der ersten Schlauchmarkierung (123, 223, 224, 325) und einer äußersten Oberfläche (122a) des ersten Schlauchendes (122, 222a) gleich einem Abstand zwischen einer äußersten Oberfläche einer Flüssigkeitsöffnung (72, 74, 76) einer ersten Infusionssetkomponente (40, 50, 60, 70) und einem harten Anschlag der Flüssigkeitsöffnung (72, 74, 76) ist, 76), wobei der harte Anschlag so konfiguriert ist, dass er die äußerste Oberfläche (122a) des ersten Schlauchendes (122, 222a) berührt und sie am weiteren Vorrücken in den Fluidanschluss (72, 74, 76) der ersten Infusionssetkomponente (40, 50, 60, 70) hindert, wobei die erste Schlauchmarkierung (123, 223, 224, 325) ein Material umfasst, das nur unter einem ultravioletten, UV-Lichtspektrum sichtbar ist, und wobei die erste Schlauchmarkierung (123, 223, 224, 325) mehrere zylindrische Bänder umfasst, die an dem ersten Schlauchende (122, 222a) angeordnet sind;
eine zweite Schlauchmarkierung (123, 223, 224, 325), die auf dem Schlauch (122, 123, 124, 222, 322) angeordnet ist, wobei die zweite Schlauchmarkierung (123, 223, 224, 325) so positioniert ist, dass ein Abstand (z2) zwischen einer Vorderkante (123a, 223a, 224a) der zweiten Schlauchmarkierung (123, 223, 224, 325) und der äußersten Oberfläche (122a) des zweiten Schlauchendes (124, 126, 222b) im Wesentlichen gleich einem Abstand zwischen einer äußersten Oberfläche einer zweiten Infusionsset-Komponentenfluidöffnung (72, 74, 76) und einem harten Anschlag der Fluidöffnung (72, 74, 76) der zweiten Infusionssetkomponente im Wesentlichen gleich ist, wobei die zweite Schlauchmarkierung (123, 223, 224, 325) ein Material umfasst, das nur unter einem bestimmten Lichtspektrum sichtbar ist, und wobei die zweite Schlauchmarkierung (123, 223, 224, 325) eine andere Anzahl zylindrischer Bänder umfasst als die erste Schlauchmarkierung (123, 223, 224, 325); und
eine Informationsmarkierung (225, 226, 227, 228), die auf dem Schlauch (122, 123, 124, 222, 322) angeordnet ist, wobei die Informationsmarkierung (225, 226, 227, 228) Material umfasst, das nur unter einem UV-Lichtspektrum sichtbar ist.

2. Schlauch (122, 123, 124, 222, 322) nach Anspruch 1, wobei die mehreren zylindrischen Bänder zwei unterschiedlich große massive Bänder umfassen.

3. Schlauch (122, 123, 124, 222, 322) nach Anspruch 1, wobei das Schlauchmarkierungsmaterial gedruckte Tinte umfasst.

4. Schlauch (122, 123, 124, 222, 322) nach Anspruch 3, wobei die gedruckte Tinte einen UV-Farbstoff umfasst.

5. Schlauch (122, 123, 124, 222, 322) nach Anspruch 3, wobei die gedruckte Tinte eine UV-Tinte umfasst.

6. Schlauch (122, 123, 124, 222, 322) nach Anspruch 1, wobei der zweite Schlauchmarkierer (123, 223, 224, 325) ein Material umfasst, das nur unter demselben UV-Lichtspektrum sichtbar ist wie der erste Schlauchmarkierer (123, 223, 224, 325).

7. Schlauch (122, 123, 124, 222, 322) nach Anspruch 1, wobei die zweite Schlauchmarkierung (123, 223, 224, 325) ein Material umfasst, das sich von dem Material der ersten Schlauchmarkierung (123, 223, 224, 325) unterscheidet, und wobei die zweite Schlauchmarkierung (123, 223, 224, 325) nur unter einem anderen spezifischen Lichtspektrum sichtbar ist als die erste Schlauchmarkierung (123, 223, 224, 325).

8. Infusionsset-Anordnung (30), umfassend:
eine Infusionskomponente (40, 50, 60, 70); und
den Schlauch (122, 123, 124, 222, 322) nach einem der vorhergehenden Ansprüche, der mit der Infusionskomponente (40, 50, 60, 70) verbunden ist.

9. Infusionsset-Anordnung (30) nach Anspruch 8, wobei das Schlauchmarkierungsmaterial entweder UV-Tinte oder UV-Farbstoff umfasst.

10. Infusionsset-Anordnung (30) nach Anspruch 8, wobei die mehreren zylindrischen Bänder des ersten Schlauches (123, 223, 224, 325) durch einen Tintenstrahldrucker gebildet werden.

11. Infusionsset-Anordnung (30) nach Anspruch 8, ferner umfassend:
eine zweite Infusionskomponente (40, 50, 60, 70), wobei die zweite Schlauchmarkierung (123, 223, 224, 325) so positioniert ist, dass ein Abstand (z2) zwischen einer Vorderkante (123a, 223a, 224a) der zweiten Schlauchmarkierung (123, 223, 224, 325) und der äußersten Oberfläche des zweiten Schlauchendes (124, 126, 222b) gleich einem Abstand zwischen einer Außenkante und einem harten Anschlag einer Fluidöffnung der zweiten Infusionskomponente (40, 50, 60, 70) ist.

12. Infusionsset-Anordnung (30) nach Anspruch 8, ferner umfassend:
eines von einem Lösungsmittel und einem Klebstoff, der ein Befestigungsmaterial umfasst, das das erste Schlauchende (122, 222a) mit der Infusionskomponente (40, 50, 60, 70) verbindet und nur im UV-Lichtspektrum sichtbar ist.

13. Infusionsset-Anordnung (30) nach Anspruch 12, wobei das Befestigungsmaterial einen UV-Farbstoff umfasst.

## Revendications

1. Tube intraveineux (122, 123, 124, 222, 322) destiné à être utilisé dans un set de perfusion (30), le tube (122, 123, 124, 222, 322) comprenant :
un corps en polymère flexible qui définit un chemin d'écoulement de fluide ;
une première extrémité de tube (122, 222a) ;
une deuxième extrémité de tube (124, 126, 222b) ;
un premier marqueur de tube (123, 223, 224, 325) disposé sur le tube (122, 123, 124, 222, 322), le premier marqueur de tube (123, 223, 224, 325) étant positionné de telle sorte qu'une distance (z1) entre un bord avant (123a, 223a, 224a) du premier marqueur de tube (123, 223, 224, 325) et d'une surface la plus extérieure (122a) de la première extrémité de tube (122, 222a) soit égale à une distance entre une surface la plus extérieure d'un raccord de fluide (72, 74, 76) d'un premier composant de set de perfusion (40, 50, 60 , 70) et une butée dure du raccord de fluide (72, 74, 76), la butée dure étant conçue pour entrer en contact avec la surface la plus extérieure (122a) de la première extrémité de tube (122, 222a) et pour la bloquer contre toute autre progression dans le raccord de fluide (72, 74, 76) du premier composant de set de perfusion (40, 50, 60, 70), le premier marqueur de tube (123, 223, 224, 325) comprenant un matériau seulement visible sous un spectre de lumière ultraviolette, UV, et le premier marqueur de tube (123, 223, 224, 325) étant constitué de plusieurs bandes cylindriques qui sont disposées à la première extrémité du tube (122, 222a) ;
un deuxième marqueur de tube (123, 223, 224, 325) disposé sur le tube (122, 123, 124, 222, 322), le deuxième marqueur de tube (123, 223, 224, 325) étant positionné de telle sorte qu'une distance (z2) entre le bord avant (123a, 223a, 224a) du deuxième marqueur de tube (123, 223, 224, 325) et la surface la plus extérieure (122a) de la deuxième extrémité de tube (124, 126, 222b) soit sensiblement égale à la distance entre la surface la plus extérieure d'un raccord de fluide (72, 74, 76) appartenant au deuxième composant du set de perfusion et une butée dure du raccord de fluide (72, 74, 76) appartenant au deuxième composant du set de perfusion, le deuxième marqueur de tube (123, 223, 224, 325) comprenant un matériau seulement visible sous un certain spectre lumineux, et le deuxième marqueur de tube (123, 223, 224, 325) comprenant un nombre de bandes cylindriques différent de celui du premier marqueur de tube (123, 223, 224, 325) ; et
un marquage informatif (225, 226, 227, 228) disposé sur le tube (122, 123, 124, 222, 322), le marquage informatif (225, 226, 227, 228) comprenant un matériau seulement visible sous un spectre de lumière UV.

2. Le tube (122, 123, 124, 222, 322) selon la revendication 1, sachant que les plusieurs bandes cylindriques comprennent deux bandes solides de tailles différentes.

3. Le tube (122, 123, 124, 222, 322) selon la revendication 1, sachant que le matériau de marqueur de tube comprend une encre d'impression.

4. Le tube (122, 123, 124, 222, 322) selon la revendication 3, sachant que l'encre d'impression comprend un colorant UV.

5. Le tube (122, 123, 124, 222, 322) selon la revendication 3, sachant que l'encre d'impression comprend une encre UV.

6. Le tube (122, 123, 124, 222, 322) selon la revendication 1, sachant que le deuxième marqueur de tube (123, 223, 224, 325) comprend un matériau seulement visible sous le même spectre de lumière UV que pour le premier marqueur de tube (123, 223, 224, 325).

7. Le tube (122, 123, 124, 222, 322) selon la revendication 1, sachant que le deuxième marqueur de tube (123, 223, 224, 325) comprend un matériau différent de celui du premier marqueur de tube (123, 223, 224, 325), et le deuxième marqueur de tube (123, 223, 224, 325) est seulement visible sous un spectre lumineux différent de celui du premier marqueur de tube (123, 223, 224, 325).

8. Ensemble de set de perfusion (30), comprenant :
un composant de perfusion (40, 50, 60, 70) ; et
le tube (122, 123, 124, 222, 322) selon l'une des revendications précédentes, qui est raccordé au composant de perfusion (40, 50, 60, 70).

9. Ensemble de set de perfusion (30) selon la revendication 8, sachant que le matériau de marqueur de tube comprend une encre ou un colorant UV.

10. L'ensemble de set de perfusion (30) selon la revendication 8, sachant que les plusieurs bandes cylindriques du premier marqueur de tube (123, 223, 224, 325) sont formées par une imprimante à jet d'encre.

11. L'ensemble de set de perfusion (30) selon la revendication 8, comprenant en outre :
un deuxième composant de perfusion (40, 50, 60, 70), le deuxième marqueur de tube (123, 223, 224, 325) étant positionné de telle sorte qu'une distance (z2) entre le bord avant (123a, 223a, 224a) du deuxième marqueur de tube (123, 223, 224, 325) et la surface la plus extérieure de la deuxième extrémité de tube (124, 126, 222b) soit égale à la distance entre un bord extérieur et une butée dure d'un raccord de fluide du deuxième composant de perfusion (40, 50, 60, 70).

12. L'ensemble de set de perfusion (30) selon la revendication 8, comprenant en outre :
un solvant ou un adhésif comprenant un matériau de fixation qui relie la première extrémité du tube (122, 222a) au composant de perfusion (40, 50, 60, 70) et qui est seulement visible sous le spectre de la lumière UV.

13. L'ensemble de set de perfusion (30) selon la revendication 12, sachant que le matériau de fixation comprend un colorant UV.
